Europäisches Patentamt

European Patent Office

Office européen des brevets

(11) Publication number: **0 249 449**
A1

## (12) EUROPEAN PATENT APPLICATION

(21) Application number: 87305108.0

(22) Date of filing: 10.06.87

(51) Int. Cl.⁴: **C 12 N 15/00**
A 61 K 39/108,
A 61 K 39/112, C 12 N 1/20
//(C12N1/20,C12R1:42),
(C12N1/20,C12R1:185)

(30) Priority: 11.06.86 AU 6359/86

(43) Date of publication of application:
16.12.87 Bulletin 87/51

(84) Designated Contracting States:
AT BE CH DE ES FR GB GR IT LI LU NL SE

(71) Applicant: ENTEROVAX RESEARCH PTY. LTD.
University of Adelaide
North Terrace Adelaide South Australia (AU)

(72) Inventor: Hone, David Michael
6 Warwick Street
Walkerville South Australia (AU)

Hackett, James Anthony
310 Alton Avenue
Magill South Australia (AU)

(74) Representative: Harding, Richard Patrick et al
Arthur R. Davies & Co. 27 Imperial Square
Cheltenham GL50 1RQ (GB)

The microorganism(s) has (have) been deposited with American type Culture Collection under number(s) 67406.

(54) Bacterial strain for live vaccines.

(57) An avirulent strain of an enterobacterium containing a galactose (gal) operon, said strain including at least one defined, non-revertible, mutation in the galE gene. The avirulent strain may be used as a precursor for a vaccine composition.

EP 0 249 449 A1

**Description**

"Bacterial Strain for Live Vaccines"

The present invention relates to the preparation and use of an avirulent bacterial strain for use in the preparation of live vaccines.

In the preparation of live vaccines, a plasmid or other cloning vector bearing a gene or genes of interest may be introduced into a host strain which will express those genes of interest. The host strain should be immunogenic and the determinants from the virulent orgainism introduced therein should have at least some prospect of also being immunogenic. The host strain should also be substantially harmless to man.

It is known in the prior art that mutations in the galE gene, the gene encoding UDP-glucose-4-epimerase, of S. typhimurium cause the strains to become sensitive to a galactose-induced lysis in vitro. By N-methyl-N'-nitroso-N-nitrosoguanidine mutagenesis Germanier isolated a stable galE derivative of S. typhi Ty2, a causative agent of typhoid fever in man. This strain (Ty21a) was sensitive to galactose-induced lysis in vitro. When administered to mice, Ty21a protected the mice against subsequent challenge with virulent Ty2. The protective immunogenicity of the Ty21a strain was attributed to the ability of the strain to synthesise "immunologically important cell wall lipopolysaccharides" in the presence of low levels of galactose, while the inability of the strain to kill mice was believed to be due to cell lysis after accumulation of galactose. These properties led to the proposal that Ty21a be used as a live oral vaccine against typhoid infection in man. Field trials using Ty21a have been conducted in Egypt where the strain was shown to be most effective (95%) in protecting children against typhoid. Here the vaccine was administered orally as a lyophilised preparation reconstituted in 20-30 ml of liquid. A further trial (in Chile) employed enteric-coated capsules of lyophilised bacteria. When children received 2 capsules within 7 days, 60% protection (over 2 years) was observed.

While Ty21a therefore shows promise as a vaccine against typhoid, strain Ty21a grows poorly. Such poor growth may possibly be due to the damage caused by an initial ultraviolet treatment to induce mutation. Moreover, in our hands, viability is significantly reduced by lyophilisation, an essential step in the preparation of a vaccine. The galE mutation in Ty21a is not defined in molecular terms, and, while revertants to galE+ have not been observed the possibility of such reversion cannot yet be excluded.

Accordingly, it is an object of the present invention to overcome, or at least alleviate, one or more of the difficulties related to the prior art.

Accordingly, in a first aspect, there is provided an avirulent strain of an enterobacterium containing a galactose (gal) operon, said strain including at least one defined, non-revertible, mutation in the galEgene.

It will be understood that the avirulent strain according to this aspect of the present invention provides the basis for improved live oral vaccines as well as a suitable host strain for the cloning of gene determinants of diseases of interest in humans and animals.

The enterobacterium may be selected from the Shigella and Salmonella strains, Salmonella typhi, Salmonella typhimurium and Salmonella dublin.

The mutation in the galE gene may be a defined insertion or defined deletion. A defined deletion is preferred.

It will be understood that for the mutation to be defined, the gal operon of the enterobacterium must be characterised. Once characterised, the deletion or insertion mutation may be undertaken utilising methods known per se.

In a preferred form of this aspect of the present invention, there is provided an avirulent strain of S. typhi inculding a defined, non-revertible deletion in the galE gene. The avirulent strain so defined, is immunogenic. The avirulent strain may be used to produce a live typhoid vaccine. The avirulent strain may be used as a host strain for the cloning of gene determinants of a disease of interest.

In a further aspect there is provided a plasmid including

    (a) a suitable plasmid cloning vector

    (b) a first cloned fragment of DNA from an enterobacterium containing the gal operon of cloning vector inserted into the plasmid.

The gal operon in the plasmid may include at least one defined, non-revertible mutation in the galE gene.

The gal operon fragment of DNA may be a segment of a Salmonella or Shigella bacterium. The fragment may be a segment of Salmonella typhimurium DNA or Salmonella typhi DNA. The mutation of the galE gene may be a defined insertion or deletion. A transposon insertion may be used. A defined deletion is preferred. The mutation should give a galE- phenotype.

The plasmid according to the present invention may include an EcoRI-HindIII clone of a segment of Salmonella typhimurium DNA which carries the entire gal operon.

As the plasmid cloning vector any suitable cloning vector may be used. A plasmid cloning vector may be selected from the plasmids pBR322, pSC101 and PUC9 is preferred. A particular plasmid of the type described above is that designated pADE107 described below, a sample of which is maintained in the culture collection of the University of Adelaide, Australia. Accordingly in a preferred aspect of the present invention, there is provided the plasmid pADE107.

Mutations of the gal operon may include a transposon insertion. A transposon Tn1725 insertion may be used. Examples of insertion mutations which provide a galE- phenotype are seen in plasmids pADE134, pADE137, pADE139 and pADE141 all described below.

The transposon Tn1725 inseritons described above may in turn be utilised to provide a plasmid inculding a defined deletion mutation in the galE gene, with the transposon insertions acting as markers for the ends of the deletion.

In a further aspect of the present invention there is provided a method of preparing a plasmid as described above which includes

    (a) providing

        (i) a first fragment of DNA from an enterobacterium containing the gal operon, and

        (ii) a suitable plasmid cloning vector

    (b) inserting the fragment of DNA into the plasmid cloning vector.

The method according to this aspect of the present invention may further include

    (c) introducing at least one defined galE mutation into the gal operon.

It will be understood that the method of preparing a plasmid so described may require a preliminary step of (a') characterising the gal operon of the enterobacterium of the interest.

For example, the gal operon of Salmonella typhimurium has not been characterised fully. The cloning and characterisation thereof is described in detail below. A similar characterisation may be undertaken for any enterobacterium of interest.

In a preferred form of this aspect of the present invention there is provided a method of preparing a plasmid as described above including a defined non-revertible deletion in the galE gene, which method includes

    (a) providing

        (i) a first and second plasmid each containing a cloned fragment containing the gal operon and including a defined insertion in the galE gene

        (ii) a suitable plasmid cloning vector

    (b) cloning the fragment to the left of the insertion in the first plasmid into the plasmid cloning vector, and

    (c) cloning the fragment to the right of the insertion in the second plasmid into the plasmid formed in step (b).

It will be understood by utilising the genetic material to the left and right of the two galE insertion mutations, the final product has the normal gal promoter (P) and normal galT and galK genes but no galE product is present.

The plasmids pADE134 and pADE137 may be used as the first and second plasmids. The plasmid cloning vector pGB2 may be utilised therein.

The final plasmid so formed is pADE161 described below, a sample of which is maintained in the culture collection of the University of Adelaide, Australia, and in the American Type Culture Collection GA1 ATCC No. 67406 Class Med. ade 161.

Accordingly in a preferred aspect of the present invention, there is provided the plasmid, pADE161. The plasmid pADE161 has an 0.4kb deletion in the galE gene.

In a further aspect of the method of preparing the plasmid may further include

    (d) providing a further fragment of DNA containing a gene of interest, and

    (e) inserting the fragment into the plasmid vector.

It will be understood that the order of steps (a), (b), (c), (d) and (e) may be reversed if desired.

It will further be understood that in this form the genetic material determinant of a pathogen of interest together with the galE− mutation may be introduced into a host strain in one step.

Accordingly in a further aspect of the present invention there is provided a method of preparing an avirulent strain of an enterobacterium containing a gal operon and including a defined non-revertible mutation in the galE gene which method includes

    (a) providing

        (i) a sample of a preselected bacterial strain, and

        (ii) a plasmid including a first cloned fragment of DNA containing a gal operon and including

    (a) a defined non-revertible mutation in the galE gene, and optionally

    (b) a fragment of DNA containing a gene of interest; and

    (b) inserting the cloned genes on the plasmid into the chromosome of the preselected bacterial strain.

The preselected bacterial strain may be selected from Salmonella and Shigella strains. Salmonella typhimurium, Salmonella typhi and Salmonella dublin have been found to be suitable.

Accordingly, in a still further aspect of the present invention there is provided an avirulent strain of an enterobacterium containing a gal operon and including

    (1) at least one defined non-revertible mutation in the galE gene and optionally

    (2) at least one further defined non-revertible attenuating mutation; and

    (3) a fragment of DNA containing a gene of interest. The gene of interest may be a determinant of a disease of interest in humans or animals.

The galK, galT and promotor of the gal operon remain intact so that the avirulent strain is otherwise substantially isogenic with the virulent parent strain. The avirulent strain so formed is immunogenic and may also be immunogenic for the disease of interest.

Diseases of interest may include those generated by any enterobacterial strain. For example, Vibrio cholerae is a strain incriminated in the disease cholera in humans. Specifically, the ompV gene which encodes an outer membrane protein has been incriminated as a gene determinant of the cholera disease in humans.

The at least one further defined non-revertible attenuating mutation may include a deletion in the Vi gene.

3

The Vi antigen is thought to be of importance in virulence of Salmonella typhi strains.

Accordingly in a preferred aspect of the present invention there is provided avirulent strains of enterobacteria selected from Salmonella typhi Ty2 strain, Ty2H1 and Ty2H1Vi−; and the Salmonella typhimurium C5 and LT2 strains C5H1 and LT2H1 as described hereinafter.

Accordingly in a still further aspect of the present invention there is provided a vaccine composition including an avirulent strain of an enterobacterium containing a gal operon and including

(1) at least one defined non-revertible mutation in the galE gene and, optionally

(2) at least one further defined, non-revertible attenuating mutation; and

(3) a fragment of DNA containing a gene of interest.

The vaccine composition may be used as a live oral vaccine against enteropathogenic diseases of man or domestic animals.

More preferably the vaccine composition includes an avirulent strain of an enterobacterium selected from the Salmonella typhi strains Ty2H1 and Ty2H1Vi−; and the Salmonella typhimurium strains C5H1 and LT2H1 as described hereinafter.

The present invention will now be more fully described with reference to the examples following. It should be understood, however, that the examples are illustrative only and should not be taken as a restriction on the generality of the invention described above. In particular, the examples describe the gal operon of S.typhimurium and S.typhi. However, the selection of any other enterobacterium such as Salmonella dublin or Shigella spp. may be made in substitution therefor.

## EXAMPLES

## MATERIALS AND METHODS

### Microbiological methods

The strains and plasmids used are shown (Table 1). Strains were usually grown in a nutrient broth (Difco, catalog no. 0003, 1.6% w/v, with NaCl 0.5% w/v). Nutrient agar plates were Difco blood base agar (Difco, catalog no. 0045) at 4% (w/v), and were supplemented with antibiotics as required. The levels used were 50 ug/ml (Ap), 25 ug/ml (Cm), 16 ug/ml (Tc). Indicator plates for galactose fermentation were nutrient agar plates (above), with 0.1% (w/v) D-galactose and 0.005% (w/v) neutral red. On these plates, gal+ strains gave red colonies while gal− strains gave yellow colonies with a surrounding clear yellow zone. Minimal galactose plates were 1% (w/v) Bacto-agar (Difco) in M9 medium but with galactose (0.1%, w/v) as sole carbon source. When these plates were used to examine the ability of Δ 4 derivates to grow on galactose, they were supplemented with tryptophan (2 ug/ml).

### Lambda methods

A gene bank of S. typhimurium LT2 DNA in the lambda cloning vector λ 1059 was the kind gift of Dr. R Maurer. Here, Sau3A-cut S. typhimurium DNA was cloned into BamHI-cut vector. To probe the bank for phage carrying gal fragments, the phage were propogated on strain RM11 on nutrient agar plates. DNA from lambda plaques was transferred to nitrocellulose. The transferred DNA was probed with nick-translated plasmid pK01 (which carries the galK gene of E. coli K-12). DNA from positive clones was prepared from large plate lysates of the clones. Hybrid phage clones which contained a functional gal operon were detected by hybrid phage complementation (lytic complementation) of the gal defect in strain Δ 4, growing on minimal galactose plates.

### DNA methods

The DNA methods used include the cutting of DNA with restriction enzymes, ligation of DNA with vector DNA with or without prior phosphatase treatment, DNA purification, DNA blotting, nick-translation, and probing.

### Immunological Methods

Female LACA mice were bred under specific pathogen-free conditions at the Waite Institute, Adelaide and allowed to acclimatize in a conventional animal house for 5-7d prior to use. Mice were housed with free access to food and water. Bacteria were administered orally or i.p. to mice by standard methods. The excision of Peyer's patches, spleens, and livers of Salmonella infected mice, and enumeration of bacteria present in these organs, was performed by standard methods.

### Other methods

The generation of Tn1725 insertion mutations in plasmids was performed. The strain rU2901, when used in a conjugation experiement at 30°C, donated the Rts1::Tn1725 to the strain carrying the plasmid of interest. After selection for Rts transfer (Cm), and curing of the R-factor at 42°C, plasmid was prepared from the exconjugant pool and re-transformed into a recipient strain, selecting Cm-resistance and the antibiotic resistance encoded by the vector. Transformants obtained here invariably had a Tn1725 insertion in the plasmid of interest.

The examination of plasmid-encoded proteins by SDS-PAGE of labeled proteins made in plasmid-containing minicells has been described.

## RESULTS

### Isolation of clones (in the lambda vector 1059) which contained S. typhimurium gal DNA

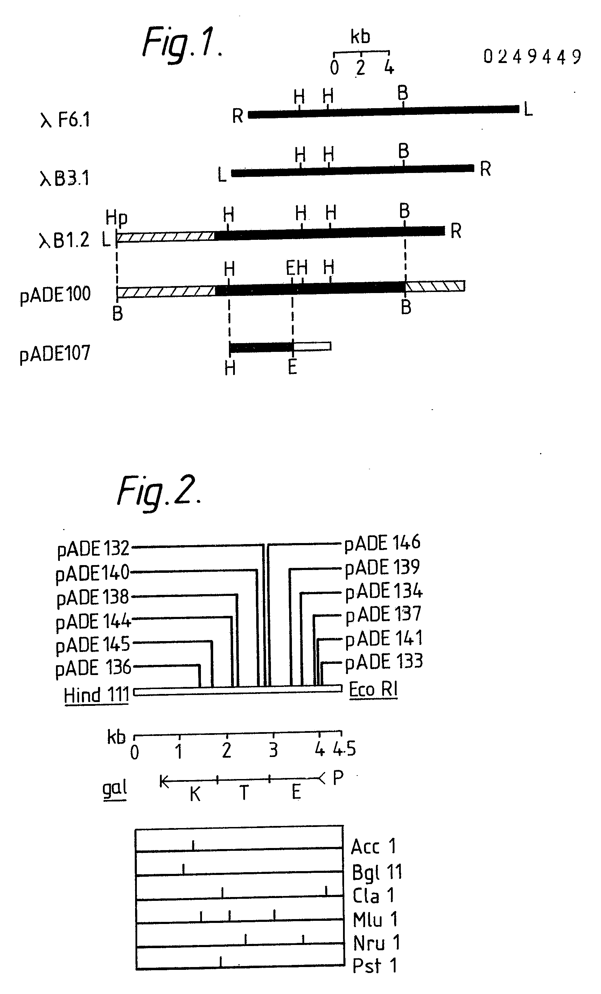

A lambda bank of S. typhimurium LT2 DNA, in the vector λ 1059,was probed with nick-translated plasmid pK01, which contained the galK gene of E. coli K12. Of about 2 x 10³ plaques screened, 3 reacted with the probe. The phage were purified and restriction enzyme analysis (Fig.1) showed that each of the three phage contained homologous cloned DNA. Lytic complementation (of the E.coli Δ gal strain Δ 4) implied that λ B1.2 (Fig. 1) contained the entire gal operon, while the other two lambda clones did not (not shown). DNA probing, using labeled pK01 as probe, showed that DNA homologour to the E.coli K12 galK gene lay on the 5.8kb HindIII fragment of λ F6.1 (Fig. 1, DNA probing not shown). Taken together, the results of the lytic complementation test and the DNA probing implied that the entire gal operon of S. typhimurium might lie in the DNA at or near the left-hand end of cloned DNA in λ B1.2 (Fig.1); DNA which included this region was subcloned into a plasmid vector.

### Subcloning of the gal operon from λ B1.2

DNA from λ B1.2 was cut with HpaI and ligated with BamHI linkers. The DNA was then cut with BamHI and ligated with phosphatase-treated BamHI-cut pJRD158B DNA. The ligation mix was transformed into E.coli K12 strain Δ 4 (Δ gal) and plated on galactose indicator plates with selection for Ap-resistance. A single gal+ transformant was obtained. Plasmid DNA (pADE100) was prepared from this strain and a partial restriction enzyme map of the insert DNA (20.5kb) obtained (Fig.1). The restriction sites for the enzyme HindIII were as expected if the cloned DNA in pADE100 had come from the region of λ B1.2 indicated in Fig. 1.

Since the insert DNA in pADE100 was 20.5kb in size, various subclones and cutdowns of the clone were prepared (Fig. 1), and examined for their ability to complement the gal deletion in strain Δ4 (Fig. 1). The plasmid pADE107 was effective in this complementation, contained only 4.5kb of cloned DNA, and was used for further study. A restriction map of the cloned DNA in this plasmid was obtained (Fig. 2).

### Localisation of the gal genes in the cloned DNA of pADE107: transposon mutagenisis

Transposon Tn1725 insertions into the cloned DNA of pADE107 were obtained and mapped (Fig. 2). Each of the plasmids carrying a Tn1725 insertion in the cloned DNA was transformed into each of E.coli K12 galE, galT, and galK strains (Table 1), and the ability of the plasmids to complement the chromosomal gal mutation was screened on indicator plates (Table 2).

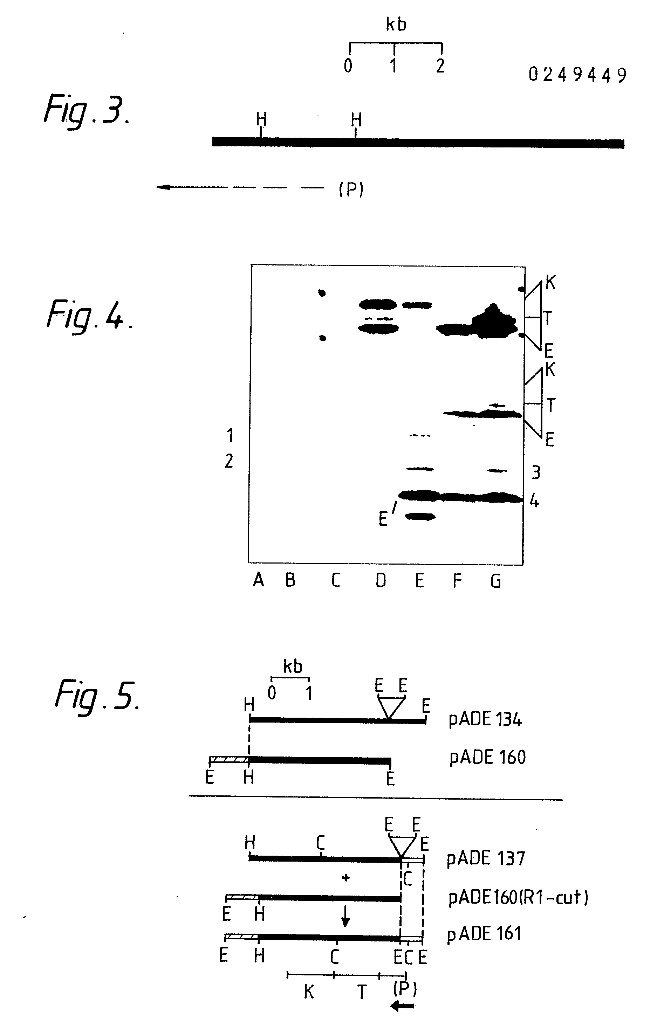

Of a total of 12 Tn1725 insertion mutations to be discussed here, one (in pADE133) did not affect the gal phenotype of the plasmid pADE107 (galE+, galT+, galK+). Four insertion mutations (in pADE134, pADE137, pADE139 and pADE141) have a galE–phenotype. With two of the mutations (in pADE137 and pADE141), the expression of the galT and galK genes was also eliminated. The other two insertion mutations (in pADE134 and pADE139) did not result in elimination of expression of the galT and galK genes. While transposon insertion mutations are generally thought to be polar, the Tn1725 may contain a promoter which reads out through an end of the transposon, as shown in Fig. 3. The direction of transcription appears to be such that transcription proceeds outward from the end of the transposon that is nearest to the transposon HindIII sites (Fig. 3). All of the "non-polar" transposon insertion mutations described in this section had the transposon inserted in this orientation upstream of the genes which were still expressed.

These results, then, indicated that the galE gene lay between the insertion point of the Tn1725 in pADE133, and extended to or past the insertion point of the transposon in pADE134. Since two of the transposon insertions generated galE mutations which were polar on galT and galK, it also seemed that these two genes did not possess their own promoters, and that the single promoter responsible for the expression of the galE, galT and galK genes in pADE107 lay near the insertion point of the transposon in pADE133.

Of the remaining 7 transposon insertion mutations to be discussed, all had a galE+ phenotype, and all lay leftward of the mutation in pADE134 (Fig. 2). five had a galT– phenotype, and of these, four were galK+. in each of these four cases, the orientation of the inserted transposon was such that galK gene expression could occur from the transposon promoter (Fig. 3). In the reamining instance (pADE146), the transposon insertion was in the opposite orientation. It appeared, then, that the galT gene extended from a point at or rightward of the pADE146 insertion (but leftward of the pADE134 insertion) to a point at or leftward of the pADE144 insertion (Fig. 2).

The last two insertions had a galE+, galT+ phenotype, but were galK– (Fig. 3). The galK gene therefore extended from a point leftward of the pADE144 insertion through to a point at or leftward of the pADE136 insertion.

In sum, the transposon insertion mutagenesis established that the gene order in the S.typhimurium gal DNA was galE, galT, galK, and that the genes appeared to constitute an operon or part of an operon, with the promoter region lying before the galE gene. The arrangement of the genes in S.typhimurium was therefore seen to be the same as that seen in E.coli K12

### Minicell analysis of the genes encoded by pADE107

Various plasmids were transformed into the minicell-producing strain DS410, and plasmid-encoded proteins labeled with [35S]-methionine and viewed on SDS-PAGE (Fig. 4). In some cases, the inducer IPTG was used (at 1mM) to activate the lac promoter of pUC9. Transcription from the lac promoter should augment the action

5

of the natural gal promoter in pADE107 (Fig. 2).

Of the several protein bands seen in Fig. 4, four are encoded by either the vector pUC9 (bands 1 and 2) or the transposon Tn1725 (bands 3 and 4). Bands 1 and 2 are seen in a strain carrying puC9 (Fig. 4, tracks A and B). Band 4 is the Cm-transacetylase enzyme of Tn1725, while band 3 is also encoded by the transposon (D. Hone, unpublished observation). The remaining four bands are labeled E, T, K and E′ (Fig. 4) and correspond to the products of the galE, galT, galK genes, and a truncated GalE protein, as explained below.

Strain DS410/pADE107 encodes the E, T and K proteins (Fig. 4, tracks C and D), the levels of which are higher if IPTG induction is employed. Band T labels poorly when compared with bands E or K. This may be due to a relatively lower methionine content of band T. The band E is not seen in DS410/pADE134 (Fig. 4, track E), but a new small band (E′) appears. As the transposon insertion in pADE134 (Fig. 2) is in the galE gene, an explanation is that band E is the galE gene product, and that band E′ is a truncated GalE protein. The RNA transcript from which this protein is translated may start at the gal promoter. Translation of this mRNA may lead to premature termination of the GalE protein at or near the site of disruption of its coding sequence by Tn1725 insertion. The Mr of E′ is about 20 kDAl (not shown). If the translation termination point were at the site of transposon insertion in pADE134, the translation start point should lie about 0.6 kb from this insertion point. As seen (Fig. 2), the gal promoter in pADE107 lies about 0.6 kb rightward of the transposon insertion point in pADE134.

The transposon insertion in pADE is non-polar on galT and galK (Table 2). Hence, it should be possible to see the galT and galK gene products in Fig. 4, track E. The band K is obvious, but band T is not visible. As mentioned, band T may be poorly labeled with methionine, and is not well seen in DS410/pADE107 unless IPTG induction is used. IPTG induction did not increase the level of expression of band K, as might be expected from the fact that the promoter active to produce band K in DS410/pADE134 was not the lac promoter, but a Tn1725 promoter. We propose that while the galT product is produced in cells harbouring pADE134, it is below the level of detection in our minicell system.

Plasmid pADE132 (galE+, galT−, galK+ - Table 2) encoded bands E and K (Fig. 4, track F). The level of band E seen was higher than that seen in DS410/pADE107, while the level of band K produced was lower. Again, band T was not obvious. Here, an explanation is that the transposon insertion in galT resulted in elimination of band T, but some expression of band K occurred due to the activity of the transposon promoter. When the expression of the galK gene is reduced, the non-induced levels of the galE (and galT, if present) gene products rise, and this would explain why band E now appeared strongly in track F.

Finally, DS410/pADE136 (galE+, galT+, galK− -Table 2) produced bands E and T in large amounts (Fig. 4, track G), No band K was seen. As explained above, the uninduced levels of the galE and galT gene products rise dramatically in a galK mutant, when compared with the galK+ parent.

It thus seemed clear that bands E, T and K were the products of the galE, galT, and galK, genes respectively. The Mr values for these proteins were respectively 37 kDAl, 40 kDAl, and 42.5 kDAl (not shown), and the DNA coding capacities required for proteins of such sizes would be about 1.1, 1.2 and 1.3 kb respectively. These values are used to show the probable termini of the genes in Fig. 2.

Preparation of plasmid with a deletion in the galE gene

Refer to Fig. 5. Two of the transposon insertions in galE are contained in plasmids with the numbers pADE134 and pADE137. The deletion was made by taking the plasmid DNA from the left end of the insert in pADE134 to the left end of the transposon, and putting this into a new plasmid vector, pGB2 (Fig. 5, top). Then this plasmid (pADE160) was used as the recipient for another piece of gal DNA, this time from pADE137. Here, the gal DNA was from the right-hand end of the insert to the right end of the (different) transposon (Fig. 5, bottom). The final plasmid, pADE161, has normal galT and galK genes, but a deletion in galE. It has the normal gal promoter (P). The galT and galK genes are therefore made normally, but no galE product is present. This is the requisite phenotype for galactose-sensitivity and consequent usefulness for vaccine.

Introduction of a deletion in the galE gene into the chromosome of Salmonella strains of interest

The general procedure is shown in Fig. 6.

First, plasmid pADE161 was introduced into the strain of interest. The strain (for example S.typhi Ty2) had a normal gal operon on the chromosome. A recombination event took place such that the gal operon with the deleted galE gene recombined onto the chromosome to give a strain with a galE deletion in the chromosome. Next, the plasmid was removed to produce the final strain.

In detail:

(1) A 10 ml. culture was inoculated with a strain as shown in Fig. 6A, and grown overnight at 37°C, with shaking. In a small number of cases, the recombination event (to give the galE chromosomal deletion) took place, and the plasmid was spontaneously lost. All later steps were designed to enrich or purify bacteria in which both these events had taken place.

(2) A 1 ml. amount of the overnight culture was inoculated into 20 ml. of fresh nutrient broth with spectinomycin (120 ug/ml). The vector (pGB2) carries a spectinomycin-resistance determinant. The culture was shaken at 37°C for about one hour. Only those bacteria carrying the spectinomycin-resistance plasmid grew, but the others were not killed. To the culture was then added ampicillin (75 ug/ml), and shaking at 37°C continued for one to two hours. Ampicillin kills only growing cells (ie: the cells carrying spectinomycin-resistance). At the end of this incubation, then, the culture was enriched for bacteria of the type sought (spectinomycin-sensitive).

(3) The remaining bacteria were pelleted in a centrifuge and spread on agar plates in the presence of Felix-O bacteriophage. This phage uses as its receptor the O-antigen of a variety of enterobacteria. GalE strains do not possess a full O-antigen and are not lysed by this phage. The plates are incubated overnight at 37°C. Next day, bacterial colonies were seen on the plate. These colonies were checked to ensure that they are, firstly, resistant to Felix-O phage, secondly, galactose-sensitive, and thirdly, spectinomycin sensitive. These were now the candidate vaccine strains.

In order to check that the required chromosomal recombination event had taken place, strains isolated as described above were examined as follows. DNA was prepared from varius strains, cut with restriction enzyme EcoRI, the fragments separated on a gel, and probed with nick- translated pADE107. The probe-reactive band pattern was viewed. Since the exact size of the deletion sought was known, and since the locality of EcoRI restriction sites in the gal DNA area was known, it was possible to show that several strains examined had the required deletion in the chromosome.

Strain Nomenclature

GalE deletions, as described above, were introduced into the chromosomes of 2 strains of S. typhimurium and one strain of S. typhi.

As the Vi antigen of S. typhi is thought to be of importance in virulence, we also made Vi– derivatives of S. typhi strains of interest. The strains to be discussed below are:

C5H1 : S. typhimurium C5, galE deletion
LT2H1 : S. typhimurium LT2, galE deletion
Ty2 : S. typhi Ty2
Ty2Vi–: S. typhi Ty2, selected Vi– by resistance to phage VilI
Ty2H1 : Ty2, galE deletion
Ty2H1Vi– :Ty2Vi– galE deletion

Physical Characteristics of the galE deletion strains

The aim of this work was the construction of a galE mutant of S. typhi Ty2 which was more vigorous in growth than Ty21a, withstood lyophilisation better, and yet was avirulent and protective in man. In this section, we examine the growth characteristics of the galE-H1 strains constructed here, their tolerance to lyophilisation, and examine the S. typhimurium galE-H1 strains for avirulence in mice and ability to protect mice against challenge with virulent S typhimurium.

As candidate avirulent vaccine strains should not revert to virulence, we examined the reversion rate of the galE-H1 strains to gal+. C5H1, LT2H1, and ty2H1 were grown for 16h at 37°C in nutrient broth (1-litre cultures). The bacteria were pelleted, washed, resuspended in saline and spread on 20x20cm plates of minimal galactose agar. The plates were incubated at 37°C for 3d. No colonies appeared on the plates. The rate of reversion of the galE-H1 mutation to gal+ was accordingly 1 in $10^{12}$.

The growth of the 3 galE-H1 strains was compared with the growth of their parent strains and Ty21a (Table 3). In shaking nutrient broth cultures at 37°C, Ty21a had a mean generation time of 65 min, whereas the other 6 strains had mean generation times of 25-30 min. Hence, the galE-H1 mutation had not affected the growth properties of the strains under these conditions.

The susceptibility of Ty21a to galactose-induced lysis in vitro (at or above 6mM galactose) is thought to be of importance in its avirulence in man in vivo. Hence we examined the effect of galactose on the growth of our galE-H1 strains and their parents. Galactose added to growing cultures to 0.2mM lysed strains with the H1 mutation, but did not affect the growth of the parent strains.

We have previously found that Ty21a does not tolerate lyophilisation well. We sought to determine if the galE-H1 strains were more resistnat to this process. Accordingly, various strains were lyophilised in nutrient broth in the presence or absence of 5% [w/v] glucose as cryoprotectant (Table 4). The recovery of Ty21a after lyophilisation was low (8% and 0.4% with or without cryoprotectant, respectively). The other strains examined (the galE-H1 strains and their parents) were better in survival. Without cryoprotectant, the lowest recovery of the 6 strains was seen with Ty2H1 (3.7%). With cryoprotectant, the lowest recovery was seen with LT2H1 (94%). There were no significant differences between the survival of galE-H1 strains and the gal+ parents.

The ability of Ty21a to effect the synthsis of smooth LPS when grown in low concentrations of galactose is thought to be important for immunogenicity of the strain in vivo. We sought evidence that the galE-H1 strains were also able to effect such synthesis. When grown in nutrient broth with both glucose (0.1% w/v), and galactose (25mM) all 3 of the galE-H1 C5H1, LT2H1 and Ty2H1 strains produced long-chain LPS. The levels of LPS synthesized were approximately the same as those made by the parent strains. The glucose was included in the galactose-containing medium as the lytic effect of galactose on galE-H1 strains is inhibited by glucose.

A virulence plasmid of about 90 kb present in S.typhimurium plays an important role in virulence. We therefore checked its presence in strains LT2H1 and C5H1. The virulence plasmid was present in all of C5, LT2, C5H1 and LT2H1 strains. No large plasmids were found in S. typhi Ty2 or strains Ty21 or Ty2H1.

Virulence and Immunogenicity of LT2H1 and C5H1

To evaluate the virulence of LT2H1 and C5H1, the median lethal dose in mice (LD50) was investigated by the oral of i.p. route of infection. In contrast to their parental strains, both LT2H1 and C5H1 were found to be completely avirulent (Table 5).

To assess the efficacy of C5H1 and LT2H1 as live oral vaccines in mice against S. typhimurium infection, groups of mice were fed with the galE-H1 strains and challenged orally with C5 14d after administration of the vaccine dose (Table 6). When mice received 2.1-2.5 x $10^8$/mouse of C5H1 or LT2H1 the $LD_{50}$ values of C5 in the immunized mice were $5 \times 10^9$ and $5 \times 10^8$ respectively, while the $LD_{50}$ value of C5 in non-immunized mic was $4.6 \times 10^4$. (Table 5).

The immunizing ability of strains C5H1 and LT2H1 was reflected in their ability to persist, following oral administration, in the Peyer's patches of the mouse intestine. While the virulent parents, C5 and LT2 increased in numbers in the Peyer's patches until death of the mice, the galE-H1 strains C5H1 and LT2H1 increased in numbers only until d3 post-administration and then declined. The establishment of such a limited infection in the Peyer's patches has been shown to be important for the generation of anit-Salmonella immunity in mice. Consistent with these results, strain C5H1 was cleared within a few days of i.p. injection into mice; by d6 after injection of $0.7 \times 10^7$ organisms/mouse, each of five mice examined had no detectable bacterial counts in peritoneum, speen or liver. In contrast, i.p. injection of $0.7 \times 10^2$ C5 organisms/mouse gave $6 - 45 \times 10^6$ organisms in each of peritoneum, liver and spleen of each of five mice at d6 post-injection.

We conclude then, that while C5H1 and LT2H1 are vigorous non-reverting strains which are tolerant of lyophilisation, they are both avirulent in mice and effective as live oral vaccines against mouse typhoid. Strain Ty2H1 was also shown to be vigorous, non-reverting, and tolerant of lyophilisation.

## Examination of virulence properties of Ty2- derived strains

As we wished to use Ty2H1 and derivatives as candidate live oral vaccines against typhoid fever in man, we sought to show that galE-H1 strains were more attenuated in various tests than their gal+ parents.

Resistance to the bactericidal action of serum is a property commonly associated with virulence. Hence, we obtained 2 sera from individuals, and adsorbed this serum with Ty2 ($10^{10}$/ml for 1h at 4°c). The adsorbed serum was tested for it bactericidal capacity (Table 7). While Ty2 was relatively serum resistant, both Ty2Vi- and the 2 galE-H1 strains examined were markedly more sensitive to serum.

The $LD_{50}$ values of the various strains in mice, after ip injection in saline or in 5% (w/v) hog gastric mucin, were obtained (Table 8). The $LD_{50}$ values for the Ty2H1 strain were not greatly increased over those for Ty2, but the introduction of the galE-H1 deletion mutation into the Ty2vi- strain resulted in greater than a 50 fold decrease in virulence, when the bacteria were administered in mucin.

We reasoned that galE-H1 strains of bacteria decreased in virulence for mice should persist in mice to a lesser extent that their more viulent parents. Hence, mice received various doses, i.p., of strains of interest, and bacterial numbers in the spleens enumerated to d15 post-injection (Table 9). While Ty2 and Ty2Vi- persisted in spleens to d15 post injection, Ty2H1 was not detectable in spleens at this time, and Ty2H1Vi-, although given at a dose approximately $10^2$-fold higher than the other strains, could never be detected in spleens.

The reduction in the ability of galE-H1 strains of S. typhi to colonise the spleens of mice when given i.p. was mirrored in the relative inability of the galE-H1 strains to persist in the mouse peritoneal cavity after injection (Table 10). While both Ty2 and Ty2Vi- persisted in detectable numbrs to 2h post injection, the galE-H1 strains did not.

These tests implied that the galE-H1 strains constructed, particularly Ty2H1Vi-, were highly attenuated for virulence in the mouse, and argued for their use as candidate live oral vaccines against typhoid fever in man.

## FIGURE LEGENDS

### Fig. 1: Cloning of the gal DNA of S.typhimurium LT2

A lambda gene bank (in λ 1059) of S.typhimurium DNA was probed with labeled pK01 DNA (which contains thegalK gene of E.coli (K12). Three gal-positive clones were detected. Recombinant DNA was prepared from the clones and restriction enzyme digests showed that the three clones had insert DNA in common - inserts are drawn to scale above and aligned in a manner which emphasises their homology. The junction points between lambda DNA and insert DNA were defined by HindIII and HpaI-HindIII digests of the clones - the lambda right arm has a HindIII site 2.0 kb from the junction point, while the lambda left arm has a HpaI site 6.9 kb from the junction point. This HpaI site is shown only for λ B1.2, as it was used in a plasmid cloning step which gave pADE100. DNA of λ B1.2 was cut with HpaI and BamHI linkers were added. Subsequently, the DNA was cut with BamHI and ligated with BamHI-cut pJRD1588 DNA to give pADE100. An EcoRI-HindIII segment of PADE100 was ligated with similarly cut pUC9 DNA to give pADE107, which contained the entrie gal operon. Not all of the restriction enzyme sites in lambda or cloned DNA are shown above - only those useful either in alignment of the cloned fragments in the 3 lambda clones, or those used in plasmid subcloning steps. L: left arm of λ 1059; R: right arm of λ 1059; B:BamHI site; E: EcoRi site; H:HindIII site; Hp:HpaI site. ▬▬▬: cloned S. typhimurium DNA; ▨▨▨: lambda DNA; ▨▨▨: pJRD1588 DNA; ▭▭▭: pUC9 DNA. A scale (kb) appears on top.

### Fig. 2: The location of the gal genes of S. typhimurium in pADE107

Plasmid pADE107 is an EcoRI-HindIII clone, in pUC9, of a 4.5 kb segment of S.typhimurium DNA which carries the gal operon (See Fig. 1 and the text). A restriction enzyme map of the cloned DNA is shown (bottom). In addition, the following enzymes do not have sites in the insert: BamHI, DraI, KpnI, PvuII, SacI, ScaI and XhoI. Transposon Tn1725 insertion mutations were generated in the cloned DNA - the positions of Tn1725 insertions were shown with the plasmid numbers of plasmids carrying Tn1725 (top). The gal phenotypes shown by these plasmids in a chromosomal Δ gal background are given in Table 2 (and see the text). These phenotypes, and analysis of proteins encoded by some of these plasmids in mincells, gabe the order to genes in the gal operon, and their sizes (middle).. P: gal promoter.

Fig. 3: A promoter in transposon Tn1725 can effect transcription out through an end of the transposon

As explained in the text, Tn1725 insertions in pADE107 were sometimes not polar on gal lying between the transposon insertion point and the galK end of the gal operon. The orientation of such transposon insertions in pADE107 was always such that the HindIII sites of the transposon lay nearer the galK end of the gal operon. Polar transposon insertions always had the other orientation. An explanation of these observations is that the transposon Tn1725 contains an internal promoter ((P) above) and that transcription from this promotor proceeds in the direction shown (from right to left, when the transposon is drawn with its HindIII sites as shown). Transcription from this promoter may also proceed out through the end of the transposon to give the observed effects on gal expression. H: HindIII site.

Fig. 4: Minicell analysis of the proteins encoded by various plasmids containing S. typhimurium gal DNA

Various plasmids were transformed into the minicell-producing strain DS410, and plasmid-encoded proteins viewed by autoradiography of an SDS-PAGE gell of total minicell proteins after [$^{35}$S]-methionine labelling of minicells purified from the various strains. The main body of the Figure is a 24 h autoradiograph, but an insert of a 72 h autoradiograph shows bands more clearly. Bands 1 and 2 are encoded by pUC9 while bands 3 and 4 are encoded by transposon Tn1725 (see the text). Bands E,T,K, and E' represent the products of the galE, galT and galK genes, and a truncated GalE protein, respectively, as explained in the text. A: DS410/pUC9; B: DS410/pUC9, IPTG-induced; C: DS410/pADE107; D: DS410/pADE107, IPTG-induced; E: DS410/pADE139; F: DS410/pADE132; G: DS410/pADE136.

Fig. 5: The construction of a plasmid containing a deletion in the galE gene of the gal operon

Plasmid pADE134 carries a Tn1725 insertion in galE (see Fig. 2). This plasmid was cut with EcoRI and HindIII and the 3.6 kb gal fragment ligated with HindIII, EcoRI-cut pGB2 DNA to give pADE160 (above the line). Next, pADE137 (Fig. 2), which carries a galE Tn1725 insertion not identical to that in pADE134 was cut with EcoRI and the smaller fragment ligated with EcoRI-cut pADE160 to give pADE161. This plasmid has the gal promoter (P), and the galT (T) and galK (K) genes, but carries a 0.3 kb deletion in the galE gene. ▬▬▬: gal DNA, ▨▨▨▨: pGB2 DNA; ▭▭▭: gal DNA from pADE137. The arrow shows the direction of promotion from promoter P.

Table 1: <u>The strains and plasmids used in this report</u>

| Strain | Genotype | Relevant Characteristic | Source |
|---|---|---|---|
| △4 | △4 (galK-attL), recA, trpC | gal-delete | Ahmed |
| KA56 | HfrH, galE45, relA1, spoT1, thi-1 λ⁻ | galE | Davey |
| CA13 | HfrH, galT118, relA1, spoT, thi-1, λ⁻ | galT | Davey |
| P1700 | thr-1, leu-6, galK2, proA lacY1, trp(UAG), his-4, non-9, rpsL31, xyl-5, mtl-1, argE3, thi-1, ara-14 | galK | Reeves |
| DS410 | minA, minB, rpsL | Minicell producer | Reeve |
| RU2901 | $R_{ts}1::Tn1725$ | Carries Tn1725 | Schmidt |
| RM11 | Not known | Host for propagation of λ 1059 gene banks | Maurer |

| Plasmid | Relevant properties |
|---|---|
| pJRD1588 | Ap, Tc-resistant |
| pUC9 | Ap-resistant, carries lac promoter |
| pKO1 | Ap-resistant, carries E.coli K12 galK gene |

Table 2: <u>The ability of transposon Tn1725 insertion mutants of the gal+ plasmid pADE107 to complement galE, galT, and galK strains of E.coli K12</u>

| Plasmid | Complementation | | |
|---|---|---|---|
| | galE | galT | galK |
| pADE107 | + | + | + |
| pADE133 | + | + | + |
| pADE137 | − | − | − |
| pADE141 | − | − | − |

| | | | |
|---|---|---|---|
| pADE134 | − | + | + |
| pADE139 | − | + | + |
| pADE132 | + | − | + |
| pADE140 | + | − | + |
| pADE138 | + | − | + |
| pADE144 | + | − | + |
| pADE146 | + | − | − |
| pADE145 | + | + | − |
| pADE136 | + | + | − |

Transposon Tn1725 insertion mutants were obtained in the cloned DNA of pADE107 (gal+). The mutated plasmids were transformed into each of galE, galT, and galK strains of E.coli K12, and the ability of the plasmids to complement the chromosomal gal lesions screened on indicator plates.
+: complementation -: no complementation. The points of transposon insertion in the various mutant plasmids are shown in Fig. 2.

Table 3:   The growth rates of strains of interest

| Strain | Generation time (min) |
|---|---|
| C5 | 25 |
| C5H1 | 27 |
| LT2 | 25 |
| LT2H1 | 30 |
| Ty2 | 25 |
| Ty2H1 | 25 |
| Ty21a | 65 |

Overnight cultures of the strains were diluted 20-fold into nutrient broth and shaken at 37°C until an $OD_{650}$ of about 0.2 was attained. The bacteria were then diluted with pre-warmed broth to an $OD_{650}$ of 0.1, and growth while shaking at 37°C was continued. Cell density measurements were taken periodically and the times taken for the cultures to reach an $OD_{650}$ value of 0.8 were divided by 3 to obtain the generation times given above.

**Table 4:  Tolerance of various strains to lyophilisation**

| Strain | % recovery + S.E. | |
|---|---|---|
| | Nutrient Broth | Nutrient Broth + 5% (w/v) glucose) |
| C5 | 6.3 ± 1.4 | 94 ± 3 |
| C5H1 | 101.1 ± 2.7 | 107 ± 4 |
| LT2 | 7.7 ± 1.4 | 96 ± 2 |
| LT2H1 | 11.4 ± 3.4 | 94 ± 1 |
| Ty2 | 5.0 ± 2.6 | 101 ± 3 |
| Ty2H1 | 3.7 ± 0.8 | 98 ± 2 |
| Ty21a | 0.4 ± 0.1 | 8 ± 2 |

Various strains were streaked onto nutrient agar plates and after 16 h at 37°C the bacteria were scraped into either nutrient broth, or nutrient broth with 5% (w/v) glucose to give a concentration of $1.01 - 2.4 \times 10^{11}$ /ml. Quantities (0.1ml) of these suspensions were lyophilised and after 24h, ampoules (3 for each strain freeze-dried in each medium) were opened and the contents resuspended in nutrient broth (10ml) for viable counts.

**Table 5:  The S. typhimurium strains C5H1 and LT2H1 are avirulent in mice**

| Strain | $LD_{50}$ Oral | IP |
|---|---|---|
| C5 | $4.6 \times 10^4$ | $< 5$ |
| LT2 | $6.2 \times 10^6$ | $4.5 \times 10^2$ |
| LT2H1 | $> 10^9$ | $> 10^7$ |
| C5H1 | $> 10^9$ | $> 10^7$ |

Mice received various oral or i.p. doses of the strains shown, and deaths to d35 post-challenge were recorded. No deaths were seen with either C5H1 or LT2H1 - the numbers in the $LD_{50}$ columns are the highest doses administered.

**Table 6: Both LT2H1 and C5H1 are effective anti-C5 live oral vaccines in mice**

|  | deaths/10 in mice immunized with | |
| --- | --- | --- |
| Challenge dose of C5 | C5H1 | LT2H1 |
| $5.4 \times 10^9$ | 3 | ND |
| $5.4 \times 10^8$ | 1 | O |
| $5.4 \times 10^7$ | 0 | 0 |
| $5.4 \times 10^6$ | 0 | ND |

ND: not done.

Groups of 10 mice were immunized orally with either C5H1 ($2.5 \times 10^8$/mouse) or LT2H1 ($2.1 \times 10^8$/mouse) and, after 14d, challenged orally with C5 as shown above. Deaths to 30d post-challenge were recorded. Control mice which were not immunised but which were challenged with either $5.4 \times 10^6$ or $5.4 \times 10^7$ C5 at the time of challenge of the immunised group, died within 12 d of challenge.

Table 7: GalE derivatives of S. typhi are more serum-sensitive than their gal± parents

Bacteria were grown in nutrient broth with 0.1 mM galactose, and $10^4$ bacteria incubated with serum, at the dilutions shown, for 1 h at 37°C in a final volume of 1ml. A 0.1ml aliquot of the mixture was then placed for bacterial counts, and the recovery expressed as a percentage of the recovery from serum-free control incubations. Two different human sera were used.

| Strain | Serum % | | | |
| --- | --- | --- | --- | --- |
|  | 90 | 30 | 10 | 3 |
|  | % resistance | | | |
| Ty2 | 2,70* | 31,100 | 100,000 | 100,100 |
| Ty2Vi⁻ | <1,<1 | <1,<1 | <1, 72 | 100,100 |
| Ty2H1 | <1,<1 | <1,<1 | <1,<1 | <1,100 |
| Ty2H1vi⁻ | <1,<1 | <1,<1 | <1,<1 | <1,<1 |

*The 2 values refer to the 2 different sera used.

Table 8: The LD50 values i.p. in mice, for S. typhi strains of interest

Groups of 10 mice received various doses of bacteria, and deaths to d6 post-injection were monitored. Bacteria were grown in nutrient broth with 0.5% (w/v) glucose and 0.1% (w/v) galactose.

|  | LD50 when injected in | |
|---|---|---|
| Strain | Saline | Mucin |
| Ty2 | $4.5 \times 10^6$ | $2.4 \times 10^4$ |
| Ty2Vi⁻ | $>10^8$ | $2.0 \times 10^6$ |
| Ty2H1 | $1.9 \times 10^7$ | $1.8 \times 10^5$ |
| Ty2H1Vi⁻ | $>10^8$ | $>10^8$ |

Table 9: Persistence of S. typhi strains in spleens after i.p. injection into mice

Bacteria were grown in nutrient broth with 0.5% (w/v) glucose and 0.1% (w/v) galactose and injected i.p. into mice. Groups of 5 mice/strain/timepoint were killed and bacterial numbers in the spleens enumerated. Counts are given as, $\log_{10}$ of organisms, $\pm$ the standard error of the mean.

| Day | Counts in spleens, of | | | |
|---|---|---|---|---|
| | Ty2 | Ty2H1 | Ty2Vi⁻ | Ty2H1Vi⁻ |
| 1 | $2.9 \pm 0.3$ | $1.7 \pm 0.2$ | $2.6 \pm 0.4$ | $< 1.4$ |
| 3 | $3.6 \pm 0.2$ | $2.0 \pm 0.1$ | $3.1 \pm 0.1$ | |
| 6 | $4.7 \pm 0.3$ | $3.3 \pm 0.1$ | $4.6 \pm 0.1$ | $< 1.4$ |
| 10 | $2.9 \pm 0.3$ | $1.5 \pm 0.1$ | $2.8 \pm 0.2$ | |
| 15 | $2.5 \pm 0.4$ | $1.4$ | $2.3 \pm 0.1$ | |

Inoculum

$6.1 \times 10^4$   $5.0 \times 10^4$   $8.2 \times 10^4$   $4.7 \times 10^6$

Table 10: Persistence of S. typhi strains in the peritoneal cavity after i.p. injection into mice

Bacterial were grown in nutrient broth with 0.5% (w/v) glucose and 0.1% (w/v) galactose and injected i.p. into groups of 5 mice. Bacterial counts in the peritoneal cavities were enumerated 2h post-injection.

| Strain | Bacteria given to mouse | % survival |
|---|---|---|
| Ty2 | $5.1 \times 10^5$ | 24.8 |
| Ty2H1 | $4.8 \times 10^5$ | $< 0.01$ |
| Ty2Vi⁻ | $5.2 \times 10^5$ | 73.3 |
| Ty2H1Vi⁻ | $5.3 \times 10^5$ | $< 0.01$ |

Finally, it is to be understood that various other modifications and/or alterations may be made without departing from the spirit of the present invention as outlined herein.

**0 249 449**

**Claims**

1. An avirulent strain of an enterobacterium containing a galactose (gal) operon, said strain including at least one defined, non-revertible, mutation in the galE gene.

2. An avirulent strain according to claim 1 wherein the defined, non-revertible mutation in the galE gene is an insertion or deletion.

3. An avirulent strain according to claim 2 wherein the enterobacterium is selected from Shigella and Salmonella strains.

4. An avirulent strain according to claim 3 wherein the enterobacterium is Salmonella strain selected from Salmonella typhi, Salmonella typhimurium and Salmonella dublin.

5. A plasmid including
(a) a suitable plasmid cloning vector
(b) a first cloned fragment of DNA from an enterobacterium containing the gal operon inserted into the plasmid cloning vector.

6. A plasmid according to claim 5 wherein the first cloned fragment of DNA is an EcoRI-HindIII clone of a segment of Salmonella DNA which carries the entire gal operon.

7. A plasmid according to claim 6 wherein the plasmid cloning vector is the plasmid pUC9.

8. A plasmid according to claim 7, plasmid pADE107 as hereinbefore described.

9. A plasmid according to claim 5 wherein the gal operon in the plasmid includes at least one defined non-revertible mutation of the galE gene.

10. A plasmid according to claim 9 wherein the gal operon includes a transposon Tn1725 insertion mutation.

11. A plasmid according to claim 10 selected from pADE134, pADE137, pADE139 and pADE141 as hereinbefore described.

12. A method of preparing a plasmid containing a gal operon which method includes
(a) providing
(i) a first fragment of DNA from an enterobacterium containing the gal operon, and
(ii) a suitable plasmid cloning vector
(b) inserting the fragment of DNA into the plasmid cloning vector, and
(c) introducing at least one defined galE mutation into the gal operon.

13. A method according to claim 12 including the preliminary step of
(a') characterising the gal operon of the enterobacterium of interest.

14. A method of preparing a plasmid according to claim 12 including a defined non-revertible deletion in the galE gene, which method includes
(a) providing
(i) a first and second plasmid each containing a cloned fragment containing the gal operon and including an insertion mutation in the galE gene
(ii) a suitable plasmid cloning vector
(b) cloning the fragment to the left of the insertion in the first plasmid into the plasmid cloning vector, and
(c) cloning the fragment to the right of the insertion in the second plasmid into the plasmid formed in step (b).

15. A method according to claim 14 wherein the first and second plasmids are the plasmids pADE134 and pADE137 and the plasmid cloning vector is plasmid pGB2.

16. A method according to claim 15 further including
(d) providing a further fragment of DNA containing a gene of interest, and
(e) inserting the fragment into the plasmid vector.

17. A plasmid pADE161 as hereinbefore described.

18. A method of preparing an avirulent strain of an enterobacterium containing a gal operon and including a defined non-revertible mutation in the galE gene which method includes
(a) providing
(i) a sample of a preselected bacterial strain, and
(ii) a plasmid including a first cloned fragment of DNA containing a gal operon and including
(a) a defined non-revertible mutation in the galE gene, and optionally
(b) a fragment of DNA containing a gene of interest;
and
(b) inserting the cloned genes on the plasmid into the chromosome of the preselected bacterial strain.

19. A method according to claim 18 wherein the preselected bacterial strain is selected from Salmonella and Shigella strains.

20. A method according to claim 19 wherein the preselected bacterial strain is a Salmonella strain selected from Salmonella typhi, Salmonella typhimurium and Salmonella dublin.

15

21. A method according to claim 20 wherein the gene of interest includes the <u>ompV</u> gene which encodes an outer membrane protein.

22. An avirulent strain of an enterobacterium containing a <u>gal</u> operon and including
   (1) at least one defined non-revertible mutation in the <u>galE</u> gene and, optionally
   (2) at least one further defined, non-revertible attenuating mutation; and
   (3) a fragment of DNA containing a gene of interest.

23. An avirulent strain according to claim 22 wherein the preselected bacterial strain is selected from <u>Salmonella</u> and <u>Shigella</u> strains.

24. An avirulent strain according to claim 23 wherein the preselected bacterial strain is a <u>Salmonella</u> strain selected from <u>Salmonella typhi</u>, <u>Salmonella typhimurium</u> and <u>Salmonella dublin</u>.

25. An avirulent strain according to claim 24 wherein the gene of interest includes the <u>ompV</u> gene which encodes an outer membrane protein.

26. An avirulent strain according to claim 25 wherein the at least one further defined non-revertible attentuating mutation includes a deletion in the <u>Vi</u> gene.

27. An avirulent strain of an enterobacterium selected from the <u>Salmonella typhi</u> strains Ty2H1 and Ty2H1Vi−; and the <u>Salmonella typhimurium</u> strains C5H1 and LT2H1, as hereinbefore described.

28. A vaccine composition including an avirulent strain of an enterobacterium containing a <u>gal</u> operon and including
   (1) at least one defined non-revertible mutation in the <u>galE</u> gene and, optionally
   (2) at least one further defined, non-revertible attenuating mutation; and
   (3) a fragment of DNA containing a gene of interest.

29. A vaccine composition according to claim 27 wherein the avirulent strain of an enterobacterium is selected from <u>Salmonella typhi</u>, <u>Salmonella typhimurium</u> and <u>Salmonella dublin</u>.

Fig.1.

kb

0 2 4

0249449

λ F6.1

λ B3.1

λ B1.2

pADE 100

pADE107

Fig.2.

Fig.3.

Fig.4.

Fig.5.

0249449

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-3 856 935 (GERMANIER) * Abstract; page 1, line 43 - page 2, line 25; claims * | 1-4 | C 12 N 15/00 A 61 K 39/108 A 61 K 39/112 C 12 N 1/20 // (C 12 N 1/20 C 12 R 1:42 ) (C 12 N 1/20 C 12 R 1:185) |
| X | WO-A-8 300 437 (FORMAL et al.) * Abstract; page 4, line 5 - page 6, line 6; claims * | 1-4 | |
| Y | | 22-25, 28,29 | |
| X | CHEMICAL ABSTRACTS, vol. 104, no. 11, 17th March 1986, page 495, abstract no. 86609f, Columbus, Ohio, US; T. YAMAMOTO et al.: "Enteroadhesion fimbriae and enterotoxin of Escherichia coli: genetic transfer to a streptomycin-resistant mutant of the galE oral-route live-vaccine Salmonella typhi Ty2la", & INFECT. IMMUN. 1985, 50(3), 925-8 * Abstract * | 1-4 | |
| Y | Idem | 22-25, 28,29 | |
| Y | EP-A-0 125 228 (PRESIDENT AND FELLOWS OF HARVARD COLLEGE) * Abstract; claims * | 22-25, 28,29 | |

TECHNICAL FIELDS SEARCHED (Int. Cl.4)

C 12 N
A 61 K

-/-

The present search report has been drawn up for all claims

| Place of search | Date of completion of the search | Examiner |
|---|---|---|
| THE HAGUE | 24-09-1987 | YEATS S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

## DOCUMENTS CONSIDERED TO BE RELEVANT

Page  2

| Category | Citation of document with indication, where appropriate, of relevant passages | Relevant to claim | CLASSIFICATION OF THE APPLICATION (Int. Cl.4) |
|---|---|---|---|
| Y | DE-A-2 843 295  (INSTITUT FÜR IMPFSTOFFE DESSAU) * Page  2, line 6 - page 4, line 16; claims * | 22-25, 28,29 | |
| A | US-A-4 550 081  (STOCKER) * Abstract; page 6, lines 1-64 * | 10 | |
| P,X | CHEMICAL ABSTRACTS, vol. 107, no. 9, 31st August 1987, page 166, abstract no. 71807g, Columbus Ohio, US; D. HONE et al.: "Construction of defined galE mutants of Salmonella for use as vaccines", & J. INFECT. DIS. 1987, 156(1), 167-74 * Whole abstract * | 1-29 | |

TECHNICAL FIELDS
SEARCHED (Int. Cl.4)

The present search report has been drawn up for all claims

| Place of search | Date of completion of the search | Examiner |
|---|---|---|
| THE HAGUE | 24-09-1987 | YEATS S.M. |